# EUROPEAN PATENT SPECIFICATION

(11) **EP 4 498 899 B1**
(45) Date of publication and mention of the grant of the patent: **02.09.2026**
(21) Application number: 23718109.4
(22) Date of filing: 23.03.2023
(51) Int. Cl.: A61B 5/00, A61B 5/024, A61B 5/08, A61B 7/04

(54) **SYSTEMS AND METHODS TO PREDICT MORTALITY RISK**
SYSTEME UND VERFAHREN ZUR VORHERSAGE DES STERBLICHKEITSRISIKOS
SYSTÈMES ET PROCÉDÉS POUR PRÉDIRE LE RISQUE DE MORTALITÉ

(30) Priority: 25.03.2022 US 202263323799 P
(43) Date of publication of application: 05.02.2025
(73) Proprietor: Cardiac Pacemakers, Inc., St. Paul, Minnesota 55112-5798 (US)
(72) Inventor: AVERINA, Viktoria A., Shoreview, Minnesota 55126 (US); WARIAR, Ramesh, Blaine, Minnesota 55449 (US); THAKUR, Pramodsingh Hirasingh, Woodbury, Minnesota 55129 (US); KWAN, Brian, Mounds View, Minnesota 55112 (US); RUBLE, Stephen B., Lino Lakes, Minnesota 55014 (US)
(74) Representative: Pfenning, Meinig & Partner mbB
(86) International application number: PCT/US2023/016008
(87) International publication number: WO 2023/183451

(56) References cited:
- EP-A1- 2 505 132
- EP-A1- 3 892 198
- WO-A1-2009/036313
- US-A1- 2017 281 097

## Description

### TECHNICAL FIELD

This document relates generally to ambulatory patient monitoring, and more particularly, but not by way of limitation, to systems and methods for predicting risk of death.

### BACKGROUND

Ambulatory medical devices (AMDs) include implantable, subcutaneous, wearable, external, or one or more other type of medical devices having sensors configured to sense physiologic signals from a patient. Detected physiologic signals can be used to determine or monitor patient status or condition, including heart failure (HF). Frequent patient monitoring, such as using one or more ambulatory medical devices, can enable early detection of worsening patient status or condition or identification of patients or groups of patients having elevated risk of future adverse events.

Document EP 2 505 132 A1 discloses systems and methods for predicting heart failure decompensation using within-patient diagnostics. A system comprises a patient device comprising: a communication module adapted to detect an alert status of each of one or more sensors; an analysis module adapted to: calculate an alert score by combining the detected alerts; and calculate a composite alert score, the composite alert score being indicative of a physiological condition and comprising a combination of two or more alert scores.

Document WO 2009/036313 A1 discloses an adherent device to monitor a patient comprising an adhesive patch to adhere to a skin of the patient. At least four electrodes are connected to the patch and capable of electrically coupling to the patient. Impedance circuitry is coupled to the at least four electrodes to measure a hydration signal of the patient. Electrocardiogram circuitry is coupled to at least two of the at least four electrodes to measure an electrocardiogram signal of the patient. An accelerometer can be mechanically coupled to the adhesive patch to generate a signal in response to at least one of an activity or a position of the patient.

### SUMMARY

The invention is defined by the appended set of claims. Any examples, aspects, embodiments and the like disclosed herein and not falling within the scope of the claims are provided for illustrative purposes.

Systems and methods to predict and trend individualized patient mortality risk are disclosed, including determining a risk value for a plurality of physiologic measures from ambulatory monitoring devices and determining a mortality risk metric indicative of a risk of patient mortality as a first weighted combination of the plurality of physiologic measures having risk values satisfying a pre-determined condition. The plurality of physiologic measures can include or be determined using two or more of impedance information, activity information, respiratory rate information, or heart sound information of the patient determined, in certain examples, using the received physiologic information from the patient.

In an example, the mortality risk metric indicative of the risk of patient mortality can be determined using a first weighted combination of different physiologic information. In certain examples, the weighting or the combination of different physiologic information can be adjusted based on a second combination of physiologic information.

In other examples, a risk of heart failure hospitalization of the patient can be determined using a second weighted combination of a plurality of physiologic measures having risk values satisfying a pre-determined condition.

The medical device system of the invention comprises a signal receiver circuit configured to receive physiologic information from a patient and an assessment circuit configured to determine a risk value for each of a plurality of physiologic measures, the plurality of physiologic measures determined using the received physiologic information, and to determine a mortality risk metric indicative of a risk of patient mortality as a weighted combination of the plurality of physiologic measures having risk values satisfying a pre-determined condition, wherein the plurality of physiologic measures include one or more of impedance information, activity information, respiratory rate information, and heart sound information.

The risk value includes a categorical risk value, the categorical risk value indicating one of a high risk or a low risk, wherein the pre-determined condition comprises a determined indicating the high risk, wherein the assessment circuit is configured to adjust a weighting or the combination of the weighted combination of physiologic measures based on the determined categorical risk values indicating the high risk.

The heart sound information comprises a combination of third heart sound (S3) information and first heart sound (S1) information.

The system further comprises that the combination of S3 information and S1 information comprises a ratio of S3/S1.

In Example 1, the system may optionally comprise wherein the assessment circuit is configured to adjust a weighting or combination of the weighted combination of the plurality of physiologic measures based on a combination of third heart sound (S3) information, the respiratory rate information, and the activity information, wherein the combination of physiologic measures includes at least two of first heart sound (S1) information, the third heart sound (S3) information, the impedance information, the respiratory rate information, or the activity information.

In Example 2, the subject matter of Example 1 may optionally comprise wherein the signal receiver circuit is configured to receive clinical information about the patient separate from the received physiologic information, the clinical information including at least one of: patient demographic information; diagnosed comorbidities; previous treatment or hospitalization; or a type of implanted device, wherein the assessment circuit is configured to determine the mortality risk metric indicative of the risk of patient mortality using the weighted combination of the plurality of physiologic measures and the received clinical information about the patient separate from the received physiologic information.

In Example 3, the subject matter of any of Examples 1-2 may optionally comprise wherein the assessment circuit is configured to determine an alert state of the patient using the determined mortality risk metric and a threshold, and to further adjust the determined mortality risk metric as a function of a time that the determined mortality risk metric is above the threshold.

In Example 4, the subject matter of any of Examples 1-3 may optionally comprise wherein the assessment circuit is configured to schedule determinations of the mortality risk metric at a default time period, and to adjust the default time period as a function of a value of the determined mortality risk metric.

In Example 5, the subject matter of any of Examples 1-4 may optionally comprise an implantable medical device comprising the assessment circuit, wherein the assessment circuit is configured to alter or adjust one or more modes or functions of the implantable medical device based on the determined mortality risk metric, wherein the one or more modes or functions includes at least one of: an active state of a sensor of the implantable medical device; a sampling frequency or resolution of a sensor of the implantable medical device; an amount of data storage of physiologic information; or a time of communication of stored information outside of the implantable medical device.

In Example 6, the subject matter of any of Examples 1-5 may optionally comprise wherein the assessment circuit is configured to determine a trend of the determined mortality risk metric over time and to provide an output of the determined mortality risk metric trend to a user interface for display to a user or to another circuit to control or adjust a process or function of the system.

In Example 7, the subject matter of any of Examples 1-6 may optionally comprise wherein the assessment circuit configured to determine the mortality risk metric indicative of the risk of patient mortality as a relative difference between respective determined mortality risk metrics at different times.

In Example 8, the subject matter of any of Examples 1-7 may optionally comprise an implantable medical device comprising the assessment circuit, wherein the assessment circuit is configured to determine an estimated remaining battery status of the implantable medical device, wherein the assessment circuit is configured to alter or adjust one or more modes or functions of the implantable medical device based on a difference between the determined mortality risk metric and the determined estimated remaining battery status of the implantable medical device.

An example (e.g., Example 9 ) of subject matter (e.g., a method) may comprise receiving, a signal receiver circuit, physiologic information from a patient, determining, using an assessment circuit, a risk value for each of a plurality of physiologic measures, the plurality of physiologic measures determined using the received physiologic information, and determining, using the assessment circuit, a mortality risk metric indicative of a risk of patient mortality using a weighted combination of the plurality of physiological measures having determined risk values satisfying a condition, wherein the plurality of physiologic measures include one or more of impedance information, activity information, respiratory rate information, and heart sound information.

In Example 10, the subject matter of Example 9 may optionally comprise wherein the risk value includes a categorical risk value, the categorical risk value indicating one of a high risk or a low risk, wherein the pre-determined condition comprises a determined indicating the high risk, wherein the method comprises adjusting a weighting or the combination of the weighted combination of physiologic measures based on the determined categorical risk values indicating the high risk.

In Example 11, the subject matter of any of Examples 9-10 may optionally comprise adjusting a weighting or combination of the weighted combination of the plurality of physiologic measures based on a combination of third heart sound (S3) information, the respiratory rate information, and the activity information, wherein the combination of physiologic measures includes at least two of first heart sound (S1) information, the third heart sound (S3) information, the impedance information, the respiratory rate information, or the activity information.

In Example 12, the subject matter of Example 11 may optionally comprise determining an alert state of the patient using the determined mortality risk metric and a threshold, wherein adjusting the weighting or combination of the weighted combination of the plurality of physiologic measures comprises as a function of a time that the determined mortality risk metric is above the threshold.

In Example 13, the subject matter of any of Examples 9-12 may optionally comprise receiving clinical information about the patient separate from the received physiologic information, the clinical information including at least one of: patient demographic information; diagnosed comorbidities; previous treatment or hospitalization; or a type of implanted device, wherein determining the mortality risk metric indicative of the risk of patient mortality includes using the weighted combination of the plurality of physiologic measures and the received clinical information about the patient separate from the received physiologic information.

An example (e.g., Example 14) of subject matter (e.g., a system) may optionally comprise a signal receiver circuit configured to receive physiologic information from a patient, including: heart sound information, including first heart sound (S1) information and third heart sound (S3) information, respiration information, including respiratory rate information, and activity information, including an indication of activity above a threshold, and an assessment circuit configured to determine a mortality risk metric using a weighted combination of physiologic information, including the third heart sound (S3) information and the first heart sound (S1) information, wherein the assessment circuit is configured to adjust a weighting or combination of the physiologic information based on a combination of the third heart sound (S3) information, the respiration information, and the activity information.

In Example 15, the subject matter of Example 14 may optionally comprise wherein the assessment circuit is configured to adjust the weighting or combination of the physiologic information to determine the mortality risk metric to include one or more of impedance information, the respiratory rate information, or the activity information based on the combination of the third heart sound (S3) information, the respiration information, and the activity information, and wherein the assessment circuit is configured to determine the mortality risk metric using the weighted combination of physiologic information and to adjust the weighting or combination of the physiologic information without using heart rate information.

In Example 16, the subject matter of any of Examples 14-15 may optionally comprise an implantable medical device comprising the assessment circuit, wherein the assessment circuit is configured to determine an estimated remaining battery status of the implantable medical device, wherein the assessment circuit is configured to alter or adjust one or more modes or functions of the implantable medical device based on a difference between the determined mortality risk metric and the determined estimated remaining battery status of the implantable medical device.

In Example 17 , subject matter (e.g., a system or apparatus) may optionally combine any portion or combination of any portion of any one or more of Examples 1-16 to comprise "means for" performing any portion of any one or more of the functions or methods of Examples 1-16, or at least one "non-transitory machine-readable medium" including instructions that, when performed by a machine, cause the machine to perform any portion of any one or more of the functions or methods of Examples 1-16.

This summary is intended to provide an overview of subject matter of the present patent application. It is not intended to provide an exclusive or exhaustive explanation of the disclosure. The detailed description is included to provide further information about the present patent application. Other aspects of the disclosure will be apparent to persons skilled in the art upon reading and understanding the following detailed description and viewing the drawings that form a part thereof, each of which are not to be taken in a limiting sense.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIGS. 1-3 illustrate example determined risks of cardiovascular death.
FIG. 4 illustrates an example method to determine a mortality risk metric indicative of a risk of patient mortality.
FIG. 5 illustrates an example system to determine a mortality risk metric indicative of a risk of patient mortality.
FIG. 6 illustrates an example patient management system and portions of an environment in which the system may operate.
FIG. 7 illustrates an example implantable medical device (IMD) electrically coupled to a heart.
FIG. 8 illustrates a block diagram of an example machine upon which any one or more of the techniques (e.g., methodologies) discussed herein may perform.

### DETAILED DESCRIPTION

Mortality and risk of a heart failure hospitalization (HFH) in patients with an implantable cardiac defibrillator (ICD) or a cardiac resynchronization therapy defibrillator (CRT-D) devices are associated with several clinical risk factors, including cardiovascular comorbidities. Previous studies have shown that a 3-month average of thoracic impedance during 6 to 9 months after implant predicted mortality after adjusting for age, sex, and the type of device treating a patient, additive and independent to a fluid index configured to determine a risk of acute decompensation using an accumulation of a difference between a daily impedance measurement and a reference impedance measurement.

The present inventors have recognized, among other things, systems and methods for predicting a risk of mortality in patients, including an improved patient-specific risk of mortality in patients with heart failure. The systems and methods disclosed herein can include a single- or multi-sensor-based mortality risk metric, automatically generated and updated at one or more regular or adjustable intervals. In certain examples, the mortality risk metric can predict a patient-specific risk of mortality (e.g., a probability of death) at different time intervals (e.g., within the next 6 months, within the next 5 years, etc.) or as a combination of a set of risks at several points in the future. Information about the mortality risk metric can be used to trigger changes in device operation, transition between different operating modes, causing alerts or notifications to be provided to an external device or process, or one or more other device adjustments to improve the performance of the device.

Determination of a patient-specific risk of mortality at different time intervals is different than a determination of a risk of worsening heart failure or a heart failure event, as heart failure events include hospitalization or treatment or intervention that do not cause or directly result in patient mortality. The time periods, the rate of change at different time intervals, and subsequent determinations each change independently to existing determinations.

Ambulatory medical devices powered by rechargeable or non-rechargeable batteries have to make certain tradeoffs between device sensing, storage, processing, and communication characteristics, such as sensing resolution, sampling frequency, the number of active sensors, the amount of stored information, processing characteristics, or communication of physiologic information outside of the device, and battery life, or particularly in the case of implantable medical devices with non-rechargeable batteries, device replacement. There is a technological problem in the art in that not all information can be stored, not all sensors can be active in a high-power or high-resolution mode, not all algorithms can be active, and not all sensed or processed information can be communicated outside of the device at all times. Technological solutions to these identified technological problems often come in the form of improvements in sensing and processing physiologic information using a device in a way that improves device efficiency, extending the lifespan of the device, or to perform new determinations using existing sensors or information in a way that was not previously known, increasing the capabilities of an existing device without adding additional hardware to the device, or requiring additional sensors or hardware to be implanted in the patient. Efficiency improvements in one area can enable additional operation in another, improving the technical capabilities of existing devices having real-world constraints.

The single- or multi-sensor-based mortality risk metric can be determined, in certain examples, using different physiologic information of the patient or various combinations thereof, such as heart sound information (e.g., S3/S1, S3, S1, etc.), respiration information (respiratory rate (RR), tidal volume (TV), rapid shallow breathing index (RSBI), etc.), activity information, impedance information, temperature information, posture information, arrhythmia information, electrolyte information, oxygen saturation information, a composite measurement, or combinations thereof. In certain examples, the single- or multi-sensor-based mortality risk metric can be a device-based metric, without input of clinical information about the patient, such as clinician diagnosis or determination of risk, patient history, patient age, comorbidities, prior hospitalization, type of implanted device, etc. In other examples, the mortality risk metric can be a combination of a device-based and clinical-based mortality risk metric, including or considering clinical information about the patient, such as clinician diagnosis or determination of risk, patient history, patient age, comorbidities, prior hospitalization, type of implanted device, patient reported outcomes (related to symptoms or quality of life), etc. In certain examples, patient history can include a previous record of diagnosis, intervention, or treatment for one or more conditions, including chronic obstructive pulmonary disease (COPD), chronic kidney disease (CKD), anemia, heart failure, etc. In an example, separate determinations can be made for different combinations of clinical information.

In certain examples, the composite measurement can include a HeartLogic^{™} index, a HeartLogic^{™} in-alert time, or one or more other composite measurements or measures thereof. The HeartLogic^{™} index is a composite measurement from multiple ambulatory sensors, including S1 and S3 heart sounds, thoracic impedance, activity information, respiration information, and nighttime heart rate (nHR), indicative of a heart failure status, a risk of a future heart failure event, or a worsening of the heart failure status or risk of heart failure event in the patient over time. The HeartLogic^{™} in-alert time is a measure of time that the HeartLogic^{™} index is above an alert threshold.

In certain examples, the HeartLogic^{™} index can be determined using different combinations or weightings of physiologic information, including one or more of S1 and S3 heart sounds, thoracic impedance, activity information, rapid shallow breathing index (RSBI), respiratory rate, and nighttime heart rate (nHR. In certain examples, the different combinations or weightings of the HeartLogic^{™} index can be adjusted or determined based on a risk stratifier. In certain examples, the risk stratifier can be determined as a different combination of physiologic information, including one or more of S3, respiratory rate, and time active (e.g., an amount of time at a specific activity level above a mean activity level of the patient or a specific threshold, etc.). If the risk stratifier is low, or below a first threshold, the HeartLogic^{™} index can be determined using a first combination of physiologic information. If the risk stratifier is high, or above a second threshold, the HeartLogic^{™} index can be determined using the first combination of physiologic information and a second combination of physiologic information, including additional information than included in the first combination. If the risk stratifier is between the first and second thresholds, the HeartLogic^{™} index can be determined using the first combination and one or more metrics or components of the second combination, or using the first combination and the second combination, but with the second combination having less weight than if the risk stratifier is above the second threshold (e.g., using less of the second combination).

In an example, the HeartLogic^{™} index and in-alert time can include detection of worsening heart failure or prediction of a physiologic event, including determination of a risk indication or stratification, such as that disclosed in the commonly assigned An et al. U.S. Patent No. 9,968,266 entitled "RISK STRATIFICATION BASED HEART FAILURE DETECTION ALGORITHM," or in the commonly assigned An et al. U.S. Patent No. 9,622,664 entitled "METHODS AND APPARATUS FOR DETECTING HEART FAILURE DECOMPENSATION EVENT AND STRATIFYING THE RISK OF THE SAME," or in the commonly assigned Thakur et al. U.S. Patent No. 10,660,577 entitled "SYSTEMS AND METHODS FOR DETECTING WORSENING HEART FAILURE," or in the commonly assigned An et al. U.S. Patent Application No. 2014/0031643 entitled "HEART FAILURE PATIENT STRATIFICATION," or in the commonly assigned Thakur et al. U.S. Patent No. 10,085,696 entitled "DETECTION OF WORSENING HEART FAILURE EVENTS USING HEART SOUNDS,".

Implantable and ambulatory medical devices frequently contain one or more accelerometer sensors and corresponding processing circuits to determine and monitor patient acceleration information, such as, among other things, cardiac vibration information associated with blood flow or movement in the heart or patient vasculature (e.g., heart sounds, cardiac wall motion, etc.), patient physical activity or position information (e.g., patient posture, activity, etc.), respiration information (e.g., respiration rate, phase, breathing sounds, etc.), etc.

Heart sounds are recurring mechanical signals associated with cardiac vibrations or accelerations from blood flow through the heart or other cardiac movements with each cardiac cycle and can be separated and classified according to activity associated with such vibrations, accelerations, movements, pressure waves, or blood flow. Heart sounds include four major features: the first through the fourth heart sounds (S1 through S4, respectively). The first heart sound (S1) is the vibrational sound made by the heart during closure of the atrioventricular (AV) valves, the mitral valve and the tricuspid valve, and the opening of the aortic valve at the beginning of systole, or ventricular contraction. The second heart sound (S2) is the vibrational sound made by the heart during closure of the aortic and pulmonary valves at the beginning of diastole, or ventricular relaxation. The third and fourth heart sounds (S3, S4) are related to filling pressures of the left ventricle during diastole. An abrupt halt of early diastolic filling can cause the third heart sound (S3). Vibrations due to atrial kick can cause the fourth heart sound (S4). Valve closures and blood movement and pressure changes in the heart can cause accelerations, vibrations, or movement of the cardiac walls that can be detected using an accelerometer or a microphone, providing an output referred to herein as cardiac acceleration information.

Respiration information can include, among other things, a respiratory rate (RR) of the patient, a tidal volume (TV) of the patient, a rapid shallow breathing index (RSBI) of the patient, or other respiratory information of the patient. The respiratory rate is a measure of a breathing rate of the patient, generally measured in breaths per minute. The tidal volume is an aggregate measure of respiration changes, such as detected using measured changes in thoracic impedance, etc. The RSBI is a measure of respiratory frequency relative tidal volume of the patient. The nHR is a measure of heart rate (HR) of the patient at night, either in relation to sensing patient sleep or using a preset or selectable time of day corresponding to patient sleep.

Physiologic metrics, as described herein, can include one or more different measures of rate, amplitude, energy, etc., of different physiologic information over one or more time periods, such as representative daily values, etc. For example, heart sound metrics can be determined for each heart sound (e.g., the first heart sound (S1) through the fourth heart sound (S4), etc.) and can include an indication of an amplitude or energy of a specific heart sound for a specific cardiac cycle, or a representation of a number of cardiac cycles of the patient over a specific time period. Daily metrics can be determined representative of an average daily value for the patient, either corresponding to a waking time or a 24-hour period, etc. Respiration metrics can include, among other things, a mean or median respiration rate, binned values of rates, and a representative value of specific rate bins, etc. Heart rate metrics can include an average nighttime heart rate, a minimum nighttime heart rate, heart rate at rest, etc.

The activity information can include an activity measurement of the patient, such as detected using an accelerometer, a posture sensor, a step counter, or one or more other activity sensors associated with an ambulatory medical device. Activity may be used to gate other physiologic measurements such as heart rate or respiration rate so that the change in these metrics with increased patient activity may be used to infer patient cardiovascular and metabolic status including measurement of oxygen consumption. The impedance information can include, among other things, thoracic impedance information of the patient, such as a measure of impedance across a thorax of the patient from one or more electrodes associated with the ambulatory medical device (e.g., one or more leads of an implantable medical device proximate a heart of the patient and a housing of the implantable medical device implanted subcutaneously at a thoracic location of the patient, one or more external leads on a body of the patient, etc.). In other examples, the impedance information can include one or more other impedance measurements associated with the thorax of the patient, or otherwise indicative of patient thoracic impedance.

The temperature information can include an internal patient temperature at an ambulatory medical device, such as implanted in the thorax of the patient, or one or more other temperature measurements made at a specific location on the patient, etc. The temperature information can be detected using a temperature sensor, such as one or more circuits or electronic components having an electrical characteristic that changes with temperature. The temperature sensor can include a sensing element located on, at, or within the ambulatory medical device configured to determine a temperature indicative of patient temperature at the location of the ambulatory medical device.

The present inventors have recognized, among other things, based on a study with more than 1500 participants, that several measurements are unexpectedly more significant predictors than other measures of patient mortality and heart failure hospitalizations. The several measurements predictive of patient mortality included a HeartLogic^{™} index, a HeartLogic^{™} in-alert time, an S3/S1 ratio, thoracic impedance, respiratory rate, rapid shallow breathing index, and activity information, after adjusting for clinical variables, such as age, gender, and comorbidities, are predictive of patient mortality. The HeartLogic^{™} index, in particular, is a better predictor of mortality than the remaining measurements, including the individual measurements that make up the HeartLogic^{™} index. Average heart rate (as a singular measure, e.g., outside of the HeartLogic^{™} index) is not a significant predictor of mortality, and S1 (as a singular measure, e.g., outside of the HeartLogic^{™} index) is not a significant predictor of heart failure hospitalization.

Specifically, the present inventors have recognized the following significant independent device-based risk factors, adjusted for clinical covariates (including age, sex, and type of device) having more significance than other measures: for mortality, high HeartLogic^{™} index, low thoracic impedance, low activity, high median respiratory rate, and high S3/S1; and for heart failure hospitalization, high HeartLogic^{™} index, low activity, and high median respiratory rate, but not thoracic impedance. For heart failure hospitalization, low thoracic impedance is not a significant independent predictor of heart failure hospitalization unless the HeartLogic^{™} index, HeartLogic^{™} in-alert time, activity, respiratory rate, and S3 are removed from consideration.

As used herein, high and low (or high, medium, and low, etc.) can be relative or categorical terms, in certain examples with respect to clinical or population values, patient-specific values (e.g., a current value with respect to a short- or long-term range of values, etc.), or combinations thereof. For example, a high value can include a value in an upper percentage (e.g., at or above an upper quartile, etc.) of values experienced by the patient over respective time periods, such as one or more of a short-term range (e.g., having a period between 1 week and 3 months, such as 1 month, etc.), a long term range (e.g., having a period greater than the short-term range, such as greater than 1 month, greater than 3 months, the last 6 months, or longer, etc.). A low value can include a value in a lower percentage (e.g., at or below a mean or median, below the upper quartile, etc.). A medium value can, in certain examples, include a value between the upper and lower quartiles or within a threshold percentage of a mean or median, etc. In other examples, values can be determined with respect to clinical or population values, in certain examples, further respective to matching patient demographics (e.g., age, sex, comorbidities, etc.) or type of medical device (e.g., CRT-D device, ICD device, etc.), etc.

For example, categorical risk values can be determined for the respective measures (e.g., a high risk, a low risk, etc.), and then a mortality risk metric or a heart failure hospitalization metric can be determined as a function of measures having a determined high risk. For example, a HeartLogic^{™} index at or above an upper quartile can be determined as a high risk (e.g., a risk score of 1) and a low impedance having a value at or below a lower quartile can be determined as a high risk (e.g., a risk score of 1). The mortality risk metric can be determined as a first function of measures having a determined high risk (e.g., as a first weighted function of the respective measures, etc.). The heart failure hospitalization risk can be determined as a second function of measures having a determined high risk (e.g., as a second weighted function of the respective measures, etc.). In certain example, the respective measures can include one or more of the HeartLogic^{™} index, thoracic impedance, activity, respiratory rate (e.g., median respiratory rate), and S3/S1, etc.

Such determinations of mortality or heart failure hospitalization risk can be used to change device behavior, trigger additional sensing, data processing, storage, or transmission, or otherwise alter one or more modes, processes, or functions of medical devices associated with such determinations. For example, determinations can require data over a substantial time period (e.g., multiple days, weeks, a month or more, etc.). Such determinations can be initially determined by the device at yearly or semi-yearly (e.g., every 6 months, every 3 months, etc.) by default, or triggered by worsening patient status or upon instruction from a clinician or caregiver, etc. In a first example, an assessment circuit can determine such mortality or heart failure hospitalization indications quarterly, consuming a default amount of device resources. If the quarterly determination exceeds one or more of a patient-specific or population threshold, the assessment circuit can alter device functionality to increase the frequency of making such determinations, increasing the use of device resources, in certain examples reducing device lifespan, but providing additional monitoring and determinations. In other examples, if a determination exceeds one or more thresholds, additional sensing can be triggered, such as enabling additional sensors, or sensing enabled sensors with a higher resolution or sampling frequency, storing more information, and communicating more information outside of the device, such as to an external programmer, or increasing the frequency of communication outside of the device, increasing the use of device resources, in certain examples reducing device lifespan, but providing additional monitoring and determinations.

In certain examples, the determinations described herein (e.g., the mortality risk metric, etc.) can include one or more determined risk curves illustrating determined risks at different time periods into the future, such as a determined risk of mortality (e.g., cardiovascular death), a determined risk of heart failure hospitalization, etc. Information about the determined risks or the determined risk curves or portions of the determined risk curves themselves can be provided to a user, such as to a patient, clinician, caregiver, etc., or can be used to make one or more device changes, such as described herein (e.g., therapies, treatments, device settings, etc.), or trigger one or more other processes or notifications, etc.

In certain examples, the mortality risk metric can be determined using a number of combined measures of received or determined physiologic information. For example, the mortality risk metric can be determined using a HeartLogic^{™} index value, including a first weighted combination of physiologic information, including a first heart sound (S1) metric, the third heart sound (S3) information, and one or more of heart rate information, impedance information, rapid shallow breathing information, and the respiratory rate information. In an example, the assessment circuit can be configured to adjust a weighting or combination of the physiologic information based on a second combination of physiologic information, including one or more of the third heart sound (S3) information, the respiration information, and the activity information.

In certain examples, the mortality risk metric can further be determined as a function of one or more values or functions of: (1) the HeartLogic^{™} index; (2) respiratory rate information (e.g., median respiratory rate); and (3) a ratio of S3 to S1 information (e.g., S3 normalized by S1), over (divided by): (1) a thoracic impedance (TI) and (2) an activity. For example, the function can be illustrated as: *M = (a*HL + b*RR + c*S3*/*S1)*/*(d*TI + e*Activity),* where *M* is the mortality risk metric, *HL* is the HeartLogic^{™} index (described above), *RR* is respiratory rate information (e.g., daily median respiratory rate), *S3*/*S1* is the S3 normalized by changes in S1, *TI* is thoracic impedance, *Activity* is commensurate with time active by the patient, and *a, b, c, d, and e* are coefficients representing weights for respective measures. The function above indicates that the mortality risk metric can be commensurate with one or more or a combination of a high values or functions (in the numerator) of HeartLogic^{™} index (e.g., a daily value or function), median respiratory rate (e.g., a daily value or function), and S3 normalized by S1 (e.g., a daily value or function), in combination with one or more of a low values or functions (in the denominator) of thoracic impedance (e.g., a daily value or function) and activity (e.g., a daily value or function).

In other examples, other measures or mathematical combinations of measures or relationships can be used to determine the mortality risk metric of the patient. In an example, the relationship to determine the mortality risk metric can be determined using statistical analysis (e.g., machine learning techniques, such as linear models (e.g., Lasso & Ridge), tree-based models (e.g., XG Boost, Cat Boost, Light GBM, random forest (RF), Decision Tree, etc.), etc.) of physiologic information, such as described herein. These models may be automatically updated over time using machine learning and artificial intelligence tools using data stored in a central repository such as LATITUDE^{™} after linkage to deidentified mortality data from government agencies (e.g. CMS, national death index) or electronic health records.

In an example, a heart failure hospitalization risk metric can be determined using a similar relationship, except that S1 and low impedance (e.g., separate from the HeartLogic^{™} index) are not significant predictors for determining a risk of future heart failure hospitalization.

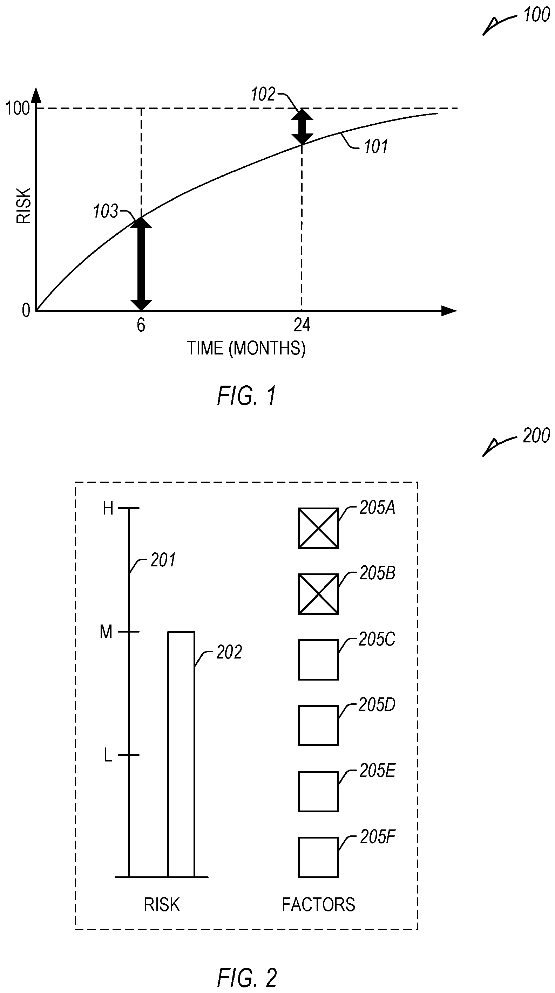

FIG. 1 illustrates an example determined risk of cardiovascular death 100 including a first example determined survival curve 101 and measures of determined risk of cardiovascular death at different time periods, including a first measure 102 at a first time period relative to the first example determined survival curve 101 (e.g., 24 months from determination of the example determined survival curve 101) and a second measure 103 at a second time period relative to the first example determined survival curve 101 (e.g., 6 months from determination of the first example determined survival curve 101). The first example determined survival curve 101 can include a determination for a patient based on one or more measurements of patient physiologic information or other information about the patient, such as described above, including patient physiologic information (e.g., cardiac electrical information, etc.), patient demographic information, diagnosis of one or more comorbidities, previous treatment or hospitalization (e.g., episodes), a type of implanted device, etc.

In an example, the determined mortality risk metric can include the determined risk of cardiovascular death, such as the first example determined survival curve 101 (e.g., in certain examples, information about the shape of the curve itself) or the measures of determined risk of cardiovascular death at different time periods. For example, the first measure 102 can indicate that a first number of patients (e.g., 10 out of 100) are likely to not experience cardiovascular death at the first time (e.g., 24 months from determination of the example determined survival curve 101) and the second measure 103 can indicate that a second number of patients (e.g., 35 out of 100) are likely to experience cardiovascular death at the second time (e.g., 6 months from determination of the example determined survival curve 101). One or more device changes or notifications can be made or determined based on information about the first example determined survival curve 101, such as to improve device performance or, in certain examples, to improve determined patient outcomes or more clearly present information for clinician review for subsequent clinical determinations.

In an example, if a determined expected risk of cardiovascular death at a specific time period (e.g., the second measure 103 at the second time period) is above a certain threshold, a notification or an alert can be provided, such as to a clinician or one or more other circuits or processes, and one or more adjustments to a device, a circuit, or a clinical workflow can be adjusted based on the determined notification or alert. In other examples, if a change or delta between expected risks of cardiovascular death determined at different times (e.g., 3 months, 6 months, 9 months, 1 year, etc.) for one or more specific time periods (such as illustrated in FIG. 1) is above a certain threshold (e.g., changes more than expected or at a faster rate than expected, etc.), the notification or alert can be provided. For example, a consultation can be scheduled with a clinician to review the determined expected risk of cardiovascular death and the information used to make such determination, a therapy can be adjusted (e.g., a medication or dosage can be changed, updated, or prescribed, etc.), one or more device settings can be changed to detect more or less information or to adjust one or more detection settings (e.g., sampling frequency, storage settings, transmission settings of detected information out of the device, etc.).

In other examples, battery life optimization of medical devices or components of medical device systems can be optimized based on the determined expected risk of cardiovascular death at a specific time period. Given a specific determined survival curve or one or more measures at one or more times relative to the determined survival curve (e.g., 24 months from determination of the determined survival curve, etc.), an expected patient end of life (EOL) can be determined, such as the number of years remaining until the determined expected risk of cardiovascular death reaches a certain threshold, such as 90%, etc. One or more functions, features, or settings of a medical device or medical device system can be adjusted to balance the use of a remaining battery life of the medical device or medical device system to benefit the patient (e.g., certain treatments, service options, avoidance of additional device replacement, sampling frequency, processing power, frequency of communication of physiologic data out of the medical device, etc.). For example, if a medical device (e.g., an implantable medical device, etc.) is in a low-power monitoring mode where certain tradeoffs are determined to extend the usable battery life of the medical device, but the estimated usable battery life of the medical device exceeds the time that the determined expected risk of cardiovascular death of the patient reaches a certain threshold (e.g., 90%, 95%, 100%, etc.), certain medical device functions can be enabled or adjusted to consume more power without negatively impacting the patient from a device replacement perspective.

For example, if the estimated remaining battery life of the medical device exceeds a determined expected patient EOL by more than a threshold (e.g., 1 year, 2 years, 1.5 times a remaining expected patient EOL, 2 times a remaining expected patient EOL, etc.), at least one or more functions, features, or settings of the medical device can be implemented that require more power from the medical device (e.g., multisite pacing versus single site pacing, more frequent monitoring or data collection, higher frequency sampling, longer sampling periods, more frequent data transmission outside of the medical device, more power intensive algorithms in the medical device, etc.). In other examples, if the estimated remaining battery life of the medical device does not exceed the determined expected patient EOL, at least one or more functions, features, or settings of the medical device can be reduced or disabled to reduce power consumption to bring the estimated remaining battery life of the medical device to be commensurate with the determined expected patient EOL, in certain examples with some remainder to reduce the need to replace the device during the life of the patient. The estimated remaining battery life of the medical device and the determined expected patient EOL can be evaluated at regular intervals (e.g., 6 months, etc.) to adjust programming of the medical device to improve medical device function and service to the patient.

In other examples, if one or more measures that indicate that a number of patients are likely to experience cardiovascular death at a certain time (e.g., the first measure 102, the second measure 103, etc.) are above a certain threshold, one or more functions, features, or settings of the medical device can be implemented that require more power form the medical device, but that provide one or more advanced screening features for the patient (e.g., heart failure screening features, etc.). For example, if the second measure 103 that indicates that a second number of patients are likely to experience cardiovascular death 6 months from determination of the example determined survival curve 101 is above a threshold (e.g., 30, 50, etc.), one or more of the following features can be enabled: left ventricular assist device (LVAD) screening; palliative care management; determination of change in medical device; increase communication frequency between medical device and external device (e.g., remote device, programmer, etc.), such as to increase the frequency of worsening heart failure monitoring, etc.; switch to a different or more power or resource intensive heart failure monitoring algorithm; more computationally intensive confirmation algorithm for estimating a determined survival curve (e.g., using additional markers such as EGM morphology, etc.); trigger a request for additional clinical factors that may improve risk calculation; switch on or off shock therapy; adjusting one or more programming features, such as AV delay, etc.; initiate cardiac contractility modulation (CCM) therapy (e.g., stimulation during contraction to improve cardiac efficiency, etc.); enable multisite pacing (MSP) in cardiac resynchronization therapy (CRT) devices (e.g., CRT-D devices, etc.); etc.

FIG. 2 illustrates an example determined risk of cardiovascular death 200 including a scale 201 illustrating relative risk of cardiovascular death (e.g., high (H), medium (M), low (L)) at a first time period (e.g., 6 months from today or determination of the risk of cardiovascular death 200) and a corresponding patient level 202 (e.g., illustrating a medium relative level, such as 50 out of 100 patients likely to experience cardiovascular death in the next 6 months). In certain examples, corresponding risk factors 205A-205F can be illustrated, highlighting factors that weighed most heavily in the determination of the level 202, such as by checking one or more of the boxes of specific corresponding risk factors 205A-205F, etc.

In FIG. 2, first and second risk factors 205A and 205B are checked as substantive risk factors (e.g., having values above or below respective thresholds). For example, the first risk factor 205A can include a HeartLogic^{™} index value greater than a first threshold (e.g., 16, such as relative to a highest value of the index of 100, etc.), and the second risk factor can include a heart sound measure greater than a second threshold (e.g., S3/S1 greater than 0.5, etc.). The third, fourth, fifth, and sixth risk factors 205C-205F include one or more other risk factors that are not checked in FIG. 2. The one or more other risk factors can include, among others, a thoracic impedance (TI) value less than a third threshold (e.g., less than 36 ohms), an activity level less than a fourth threshold (e.g., less than 0.6 hours per day), a respiratory rate (RR) greater than a fifth threshold (e.g., greater than 19 breaths per minute), or a number of ventricular events greater than a sixth threshold (e.g., a number of detected ventricular tachycardia or fibrillation events, shocks, ATP, etc., above a threshold, etc.), etc.

In other examples, one or more determined risks of cardiac death or other determinations can be provided in coarser or different terms. However, visual presentation of the relative measure can aid interpretation and understanding on the underlying determinations. In certain examples, individual sensor readings or indications of the determined risk can be transmitted to an assessment circuit, a therapy circuit, a therapy device, a wearable device, or one or more components of an external system, in certain examples, to be combined with one or more other determinations, such as a score of the Seattle Heart Failure Model (SHFM) or one or more clinical diagnoses or determinations.

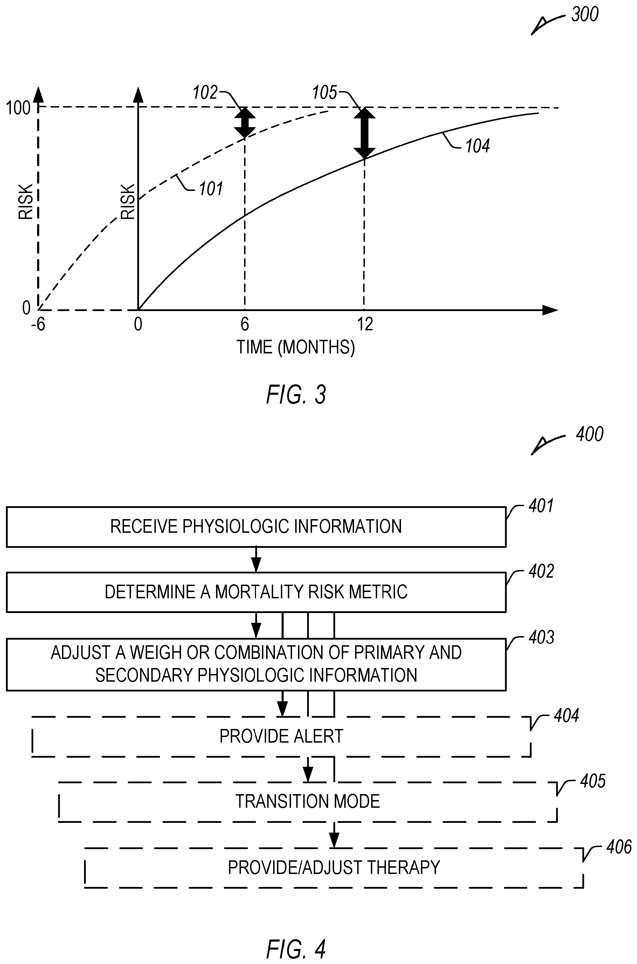

FIG. 3 illustrates an example determined risk of cardiovascular death 300, for example, including a first example determined survival curve 101 determined at a first time (e.g., -6 months) and a first measure 102 of determined risk of cardiovascular death at a first time period relative to the first example determined survival curve 101 (e.g., 12 months from determination of the first example determined survival curve 101) and a second example determined survival curve 104 determined at a second time (0 months) and a third measure 105 of determined risk of cardiovascular death at a third time period relative to the second example determined survival curve 104 (e.g., 12 months from determination of the second example determined survival curve 104).

Impact of device changes or therapy (e.g., medication, surgery, CRT, adjustment of a previous therapy, etc.), can be determined or assessed as a relative improvement in determined risk of cardiovascular death. For example, a therapy (e.g., a new therapy or therapy adjustment) can be implemented for a patient at time -6 months. The first example determined survival curve 101 can represent the determined risk of cardiovascular death for the patient at the first time, at or about the time of the therapy (e.g., just prior), and can indicate the estimated survival of the patient prior to the therapy. In the example illustrated in FIG. 3, the first measure 102 can indicate that at the first time, 10 out of 100 patients were expected to survive at 12 months (one year) prior to the therapy. The second example determined survival curve 104 can represent the determined risk of cardiovascular death for the patient at the second time, 6 months after the therapy, and can indicate the estimated survival of the patient in response to the therapy. The third measure 105 can indicate that at the second time, 20 out of 100 patients are expected to survive at 12 months (one year) after the therapy. A change between the first and third measures 102, 105 can provide an indication of therapy efficacy or patient health status.

An improvement from the first measure 102 to the third measure 105 (or between a corresponding time period on the first example determined survival curve 101 corresponding to the time of the third measure 105) can indicate a positive patient response to the therapy. If the third measure 105 does not show improvement, a notification or alert can be provided, in certain examples, triggering one or more additional therapies (or adjustment of a previous therapy), etc.

In certain examples, directional changes for key sensor readings, such as those illustrated by the corresponding risk factors 205A-205F in FIG. 2, etc., over one or more time periods (e.g., 3 months, 6 months, etc.) can be used to provide one or more adjustments to a previously implemented or existing therapy (e.g., titrate or adjust CRT therapy, titrate medication, etc.).

In other examples, contrasts or updates at different time periods can be provided or displayed in one or more other ways, such as using a format similar to that illustrated in FIG. 2, with additional corresponding patient levels (e.g., similar to the corresponding patient level 202 in FIG. 2) having different time indications (e.g., a first indicating before a new therapy or at a previous determination and a second indicating after the new therapy or at a current or more current determination than the previous determination, etc.).

Although described herein with respect to determined risk of cardiovascular death, in other examples, similar techniques can be implemented with respect to determined risks of hospitalization, events (e.g., including unexpected or unscheduled treatment or intervention), healthcare utilization (e.g., with respect to triaging an existing patient load, expected costs, etc.), or physician-initiated risk calculation providing a desired clinical or patient impact with respect to particular physicians, etc.

FIG. 4 illustrates an example method 400 to determine a mortality risk metric indicative of a risk of patient mortality. At step 401, physiologic information of a patient can be received, such as using a signal receiver circuit of an ambulatory medical device, a medical device programmer, or one or more other implantable, external, ambulatory, or remote medical devices, such as disclosed herein. In an example, the signal receiver circuit can receive the physiologic information from one or more sensors. In other examples, the signal receiver circuit can include one or more sensors configured to sense physiologic information of the patient. The physiologic information can include, in certain examples, one or more of: heart sound information, such as third heart sound (S3) information, first heart sound (S1) information, or combinations thereof; respiratory information, such as respiratory rate information, tidal volume, rapid shallow breathing index (RSBI) information, etc.; activity information, such as patient time active above a threshold (e.g., a number of hours of a day with an activity value over a threshold, etc.); impedance information, such as thoracic impedance (TI) information; etc.

At step 402, a mortality risk metric can be determined for the patient, such as using an assessment circuit. The mortality risk metric can be determined using a first combination of physiologic information, such as described above with respect to the HeartLogic^{™} index or one or more other functions above.

At step 403, a weight or combination of the first combination of physiologic information can be adjusted as a function of a second combination of physiologic information, including one or more of third heart sound (S3) information, respiratory rate information, and activity information. For example, if an initial risk determination is above one or more thresholds, indicating a high, low, or one or more other intermediate values with respect to such one or more thresholds, additional sensors or physiologic information can be additionally added to a function used to determine the mortality risk metric. In certain examples, a first set of physiologic information is used for all determinations, while a second set of physiological information is added depending on the determined risk or a previous determination of the mortality risk metric itself (e.g., if a previous determination of the mortality risk metric has a value above a threshold, additional physiologic information can be used in a subsequent determination). In certain examples, physiologic information not used in respective determinations does not have to be sensed, such that certain sensors can be disabled when not needed, saving power, but reducing sensitivity or specificity of the determined metric. In other examples, certain information may be sensed or received but not used in the determination (e.g., setting a variable to 0 in a function, etc.).

At step 404, an alert can be provided, such as by the assessment circuit, if the determined mortality risk metric exceeds a threshold, or if a difference between determined mortality risk metrics at different times exceeds a threshold or expected value. In an example, an output can be provided of the determined mortality risk metric to a user interface for display to a user or to another circuit to control or adjust a process or a function of a medical device system.

At step 405, one or more modes or functions of the assessment circuit or a medical device or medical device system can be adjusted based the determined mortality risk metric. For example, if one or more measures of determined risk of cardiovascular death indicate an expected patient end of life (EOL) longer than the determined estimated remaining battery status of a medical device (e.g., an implantable medical device), one or more modes or functions of the medical device can be altered to increase the remaining battery status of the medical device. If the determined mortality risk metric indicates that the expected patient EOL is shorter than the determined estimated remaining battery status of the medical device, one or more modes or functions of the medial device can be altered to improve data collection or sensing or to otherwise provide more patient benefit, reducing the remaining battery status of the medical device, but not shorter than the expected patient EOL, in certain examples, with additional tolerance. For example, the mode or function can include, among others, one or more of: increase communication frequency between medical device and external device (e.g., remote device, programmer, etc.), such as to increase the frequency of worsening heart failure monitoring, etc.; switch to a different or more power or resource intensive heart failure monitoring algorithm; more computationally intensive confirmation algorithm for estimating a determined survival curve (e.g., using additional markers such as EGM morphology, etc.); etc.

At step 406, one or more therapies can be provided or adjusted based on the determined mortality risk metric, including, in certain examples, one or more of: medication; surgery; left ventricular assist device (LVAD) screening; palliative care management; determination of change in medical device; trigger a request for additional clinical factors that may improve risk calculation; switch on or off shock therapy; adjusting one or more programming features, such as AV delay, etc.; initiate cardiac contractility modulation (CCM) therapy (e.g., stimulation during contraction to improve cardiac efficiency, etc.); enable multisite pacing (MSP) in cardiac resynchronization therapy (CRT) devices (e.g., CRT-D devices, etc.); etc.

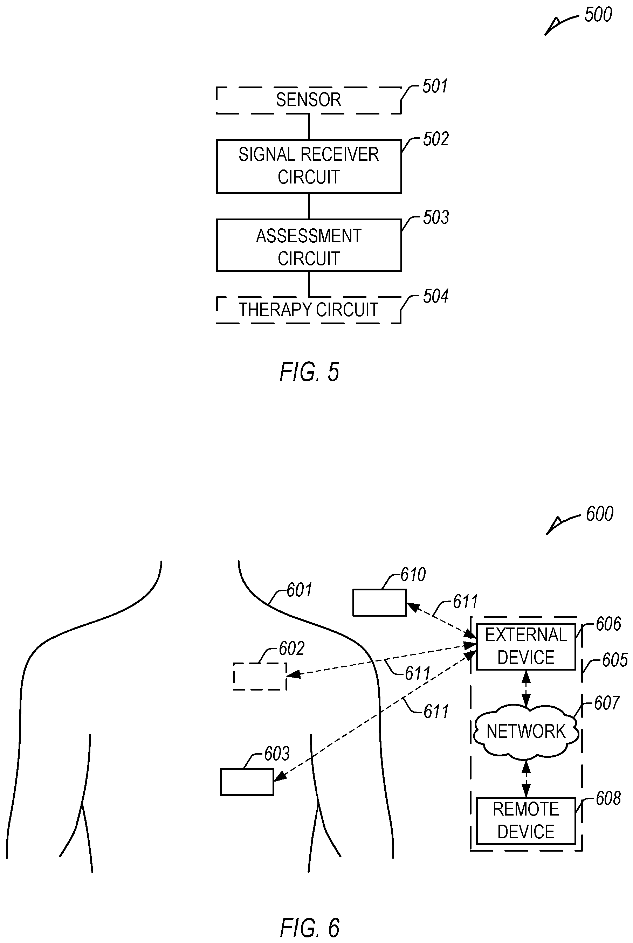

FIG. 5 illustrates an example system 500 to determine a mortality risk metric indicative of a risk of patient mortality, such as a medical-device system, a cardiac rhythm management (CRM) device, etc. In an example, one or more aspects of the example system 500 can be a component of, or communicatively coupled to, an ambulatory medical device (AMD), an insertable cardiac monitor, etc. The system 500 can be configured to monitor, detect, or treat various physiologic conditions of the body, such as cardiac conditions associated with a reduced ability of a heart to sufficiently deliver blood to a body, including heart failure, arrhythmias, dyssynchrony, etc., or one or more other physiologic conditions and, in certain examples, can be configured to provide electrical stimulation or one or more other therapies or treatments to the patient.

The system 500 can include a single medical device or a plurality of medical devices implanted in a patient's body or otherwise positioned on or about the patient to monitor patient physiologic information of the patient using one or more optional sensors, such as a sensor 501. In an example, the sensor 501 can include one or more of: a respiration sensor configured to receive respiration information (e.g., a respiration rate, a respiration volume (tidal volume), etc.); an acceleration sensor (e.g., an accelerometer, a microphone, etc.) configured to receive cardiac acceleration information (e.g., cardiac vibration information, pressure waveform information, heart sound information, endocardial acceleration information, acceleration information, activity information, posture information, etc.); an impedance sensor (e.g., intrathoracic impedance sensor, transthoracic impedance sensor, etc.) configured to receive impedance information, a cardiac sensor configured to receive cardiac electrical information; an activity sensor configured to receive information about a physical motion (e.g., activity, steps, etc.); a posture sensor configured to receive posture or position information; a pressure sensor configured to receive pressure information; a plethysmograph sensor (e.g., a photoplethysmography sensor, etc.); a chemical sensor (e.g., an electrolyte sensor, a pH sensor, an anion gap sensor, etc.); a temperature sensor; a skin elasticity sensor, or one or more other sensors configured to receive physiologic information of the patient.

The example system 500 can include a signal receiver circuit 502 and an assessment circuit 503. The signal receiver circuit 502 can be configured to receive physiologic information of a patient (or group of patients) from the sensor 501. The assessment circuit 503 can be configured to receive information from the signal receiver circuit 502, and to determine one or more parameters (e.g., physiologic parameters, stratifiers, etc.) or existing or changed patient conditions (e.g., indications of patient dehydration, respiratory condition, cardiac condition (e.g., heart failure, arrhythmia), sleep disordered breathing, etc.) using the received physiologic information, such as described herein. The physiologic information can include, among other things, cardiac electrical information, impedance information, respiration information, heart sound information, activity information, posture information, temperature information, or one or more other types of physiologic information.

The assessment circuit 503 can be configured to provide an output to a user, such as to a display or one or more other user interface, the output including a score, a trend, an alert, or other indication. In other examples, the assessment circuit 503 can be configured to provide an output to another circuit, machine, or process, such as an optional therapy circuit 504 (e.g., a cardiac resynchronization therapy (CRT) circuit, a chemical therapy circuit, etc.), etc., to control, adjust, or cease a therapy of a medical device, a drug delivery system, etc., or otherwise alter one or more processes or functions of one or more other aspects of a medical-device system, such as one or more cardiac resynchronization therapy parameters, drug delivery, dosage determinations or recommendations, etc. In an example, the therapy circuit 504 can include one or more of a stimulation control circuit, a cardiac stimulation circuit, a neural stimulation circuit, a dosage determination or control circuit, etc. In other examples, the therapy circuit 504 can be controlled by the assessment circuit 503, or one or more other circuits, etc.

Traditional cardiac rhythm management devices, such as insertable cardiac monitors, pacemakers, defibrillators, or cardiac resynchronizers, include implantable or subcutaneous devices having hermetically sealed housings configured to be implanted in a chest of a patient. The cardiac rhythm management device can include one or more leads to position one or more electrodes or other sensors at various locations in or near the heart, such as in one or more of the atria or ventricles of a heart, etc. Accordingly, cardiac rhythm management devices can include aspects located subcutaneously, though proximate the distal skin of the patient, as well as aspects, such as leads or electrodes, located near one or more organs of the patient. Separate from, or in addition to, the one or more electrodes or other sensors of the leads, the cardiac rhythm management device can include one or more electrodes or other sensors (e.g., a pressure sensor, an accelerometer, a gyroscope, a microphone, etc.) powered by a power source in the cardiac rhythm management device. The one or more electrodes or other sensors of the leads, the cardiac rhythm management device, or a combination thereof, can be configured detect physiologic information from the patient, or provide one or more therapies or stimulation to the patient.

Implantable devices can additionally or separately include one or more leadless cardiac pacemaker (LCP) devices, small (e.g., smaller than traditional implantable cardiac rhythm management devices, in certain examples having a volume of about 1 cc, etc.), self-contained devices including one or more sensors, circuits, or electrodes configured to monitor physiologic information (e.g., heart rate, etc.) from, detect physiologic conditions (e.g., tachycardia) associated with, or provide one or more therapies or stimulation to the heart without traditional lead or implantable cardiac rhythm management device complications (e.g., required incision and pocket, complications associated with lead placement, breakage, or migration, etc.). In certain examples, a leadless cardiac pacemaker can have more limited power and processing capabilities than a traditional cardiac rhythm management device; however, multiple leadless cardiac pacemakers can be implanted in or about the heart to detect physiologic information from, or provide one or more therapies or stimulation to, one or more chambers of the heart. The multiple leadless cardiac pacemaker can communicate between themselves, or one or more other implanted or external devices.

FIG. 6 illustrates an example patient management system 600 and portions of an environment in which the patient management system 600 may operate. The patient management system 600 can perform a range of activities, including remote patient monitoring and diagnosis of a disease condition. Such activities can be performed proximal to a patient 601, such as in a patient home or office, through a centralized server, such as in a hospital, clinic, or physician office, or through a remote workstation, such as a secure wireless mobile computing device.

The patient management system 600 can include one or more ambulatory medical devices, an external system 605, and a communication link 611 providing for communication between the one or more ambulatory medical devices and the external system 605. The one or more ambulatory medical devices can include an implantable medical device 602, a wearable medical device 603, or one or more other implantable, leadless, subcutaneous, external, wearable, or ambulatory medical devices configured to monitor, sense, or detect information from, determine physiologic information about, or provide one or more therapies to treat various conditions of the patient 601, such as one or more cardiac or non-cardiac conditions (e.g., dehydration, sleep disordered breathing, etc.).

In an example, the implantable medical device 602 can include one or more traditional cardiac rhythm management devices implanted in a chest of a patient, having a lead system including one or more transvenous, subcutaneous, or non-invasive leads or catheters to position one or more electrodes or other sensors (e.g., a heart sound sensor) in, on, or about a heart or one or more other position in a thorax, abdomen, or neck of the patient 601. In another example, the implantable medical device 602 can include a monitor implanted, for example, subcutaneously in the chest of patient 601, the implantable medical device 602 including a housing containing circuitry and, in certain examples, one or more sensors, such as a temperature sensor, etc.

The implantable medical device 602 can include an assessment circuit configured to detect or determine specific physiologic information of the patient 601, or to determine one or more conditions or provide information or an alert to a user, such as the patient 601 (e.g., a patient), a clinician, or one or more other caregivers or processes. The implantable medical device 602 can alternatively or additionally be configured as a therapeutic device configured to treat one or more medical conditions of the patient 601. The therapy can be delivered to the patient 601 via the lead system and associated electrodes or using one or more other delivery mechanisms. The therapy can include delivery of one or more drugs to the patient 601, such as using the implantable medical device 602 or one or more of the other ambulatory medical devices, etc. In some examples, therapy can include cardiac resynchronization therapy for rectifying dyssynchrony and improving cardiac function in heart failure patients. In other examples, the implantable medical device 602 can include a drug delivery system, such as a drug infusion pump to deliver drugs to the patient for managing arrhythmias or complications from arrhythmias, hypertension, or one or more other physiologic conditions. In other examples, the implantable medical device 602 can include one or more electrodes configured to stimulate the nervous system of the patient or to provide stimulation to the muscles of the patient airway, etc.

The wearable medical device 603 can include one or more wearable or external medical sensors or devices (e.g., automatic external defibrillators (AEDs), Holter monitors, patch-based devices, smart watches, smart accessories, wrist- or finger-worn medical devices, such as a finger-based photoplethysmography sensor, etc.).

The external system 605 can include a dedicated hardware/software system, such as a programmer, a remote server-based patient management system, or alternatively a system defined predominantly by software running on a standard personal computer. The external system 605 can manage the patient 601 through the implantable medical device 602 or one or more other ambulatory medical devices connected to the external system 605 via a communication link 611. In other examples, the implantable medical device 602 can be connected to the wearable medical device 603, or the wearable medical device 603 can be connected to the external system 605, via the communication link 611. This can include, for example, programming the implantable medical device 602 to perform one or more of acquiring physiologic data, performing at least one self-diagnostic test (such as for a device operational status), analyzing the physiologic data, or optionally delivering or adjusting a therapy for the patient 601. Additionally, the external system 605 can send information to, or receive information from, the implantable medical device 602 or the wearable medical device 603 via the communication link 611. Examples of the information can include real-time or stored physiologic data from the patient 601, diagnostic data, such as detection of patient hydration status, hospitalizations, responses to therapies delivered to the patient 601, or device operational status of the implantable medical device 602 or the wearable medical device 603 (e.g., battery status, lead impedance, etc.). The communication link 611 can be an inductive telemetry link, a capacitive telemetry link, or a radiofrequency (RF) telemetry link, or wireless telemetry based on, for example, "strong" Bluetooth or IEEE 802.11 wireless fidelity "Wi-Fi" interfacing standards. Other configurations and combinations of patient data source interfacing are possible.

The external system 605 can include an external device 606 in proximity of the one or more ambulatory medical devices, and a remote device 608 in a location relatively distant from the one or more ambulatory medical devices, in communication with the external device 606 via a communication network 607. Examples of the external device 606 can include a medical device programmer. The remote device 608 can be configured to evaluate collected patient or patient information and provide alert notifications, among other possible functions. In an example, the remote device 608 can include a centralized server acting as a central hub for collected data storage and analysis. The server can be configured as a uni-, multi-, or distributed computing and processing system. The remote device 608 can receive data from multiple patients. The data can be collected by the one or more ambulatory medical devices, among other data acquisition sensors or devices associated with the patient 601. The server can include a memory device to store the data in a patient database. The server can include an alert analyzer circuit to evaluate the collected data to determine if specific alert condition is satisfied. Satisfaction of the alert condition may trigger a generation of alert notifications, such to be provided by one or more human-perceptible user interfaces. In some examples, the alert conditions may alternatively or additionally be evaluated by the one or more ambulatory medical devices, such as the implantable medical device. By way of example, alert notifications can include a Web page update, phone or pager call, E-mail, SMS, text or "Instant" message, as well as a message to the patient and a simultaneous direct notification to emergency services and to the clinician. Other alert notifications are possible. The server can include an alert prioritizer circuit configured to prioritize the alert notifications. For example, an alert of a detected medical event can be prioritized using a similarity metric between the physiologic data associated with the detected medical event to physiologic data associated with the historical alerts.

The remote device 608 may additionally include one or more locally configured clients or remote clients securely connected over the communication network 607 to the server. Examples of the clients can include personal desktops, notebook computers, mobile devices, or other computing devices. System users, such as clinicians or other qualified medical specialists, may use the clients to securely access stored patient data assembled in the database in the server, and to select and prioritize patients and alerts for health care provisioning. In addition to generating alert notifications, the remote device 608, including the server and the interconnected clients, may also execute a follow-up scheme by sending follow-up requests to the one or more ambulatory medical devices, or by sending a message or other communication to the patient 601 (e.g., the patient), clinician or authorized third party as a compliance notification.

The communication network 607 can provide wired or wireless interconnectivity. In an example, the communication network 607 can be based on the Transmission Control Protocol/Internet Protocol (TCP/IP) network communication specification, although other types or combinations of networking implementations are possible. Similarly, other network topologies and arrangements are possible.

One or more of the external device 606 or the remote device 608 can output the detected medical events to a system user, such as the patient or a clinician, or to a process including, for example, an instance of a computer program executable in a microprocessor. In an example, the process can include an automated generation of recommendations for anti-arrhythmic therapy, or a recommendation for further diagnostic test or treatment. In an example, the external device 606 or the remote device 608 can include a respective display unit for displaying the physiologic or functional signals, or alerts, alarms, emergency calls, or other forms of warnings to signal the detection of arrhythmias. In some examples, the external system 605 can include an external data processor configured to analyze the physiologic or functional signals received by the one or more ambulatory medical devices, and to confirm or reject the detection of arrhythmias. Computationally intensive algorithms, such as machine-learning algorithms, can be implemented in the external data processor to process the data retrospectively to detect cardia arrhythmias.

Portions of the one or more ambulatory medical devices or the external system 605 can be implemented using hardware, software, firmware, or combinations thereof. Portions of the one or more ambulatory medical devices or the external system 605 can be implemented using an application-specific circuit that can be constructed or configured to perform one or more functions or can be implemented using a general-purpose circuit that can be programmed or otherwise configured to perform one or more functions. Such a general-purpose circuit can include a microprocessor or a portion thereof, a microcontroller or a portion thereof, or a programmable logic circuit, a memory circuit, a network interface, and various components for interconnecting these components. For example, a "comparator" can include, among other things, an electronic circuit comparator that can be constructed to perform the specific function of a comparison between two signals or the comparator can be implemented as a portion of a general-purpose circuit that can be driven by a code instructing a portion of the general-purpose circuit to perform a comparison between the two signals. "Sensors" can include electronic circuits configured to receive information and provide an electronic output representative of such received information.

The therapy device 610 can be configured to send information to or receive information from one or more of the ambulatory medical devices or the external system 605 using the communication link 611. In an example, the one or more ambulatory medical devices, the external device 606, or the remote device 608 can be configured to control one or more parameters of the therapy device 610. The external system 605 can allow for programming the one or more ambulatory medical devices and can receives information about one or more signals acquired by the one or more ambulatory medical devices, such as can be received via a communication link 611. The external system 605 can include a local external implantable medical device programmer. The external system 605 can include a remote patient management system that can monitor patient status or adjust one or more therapies such as from a remote location.

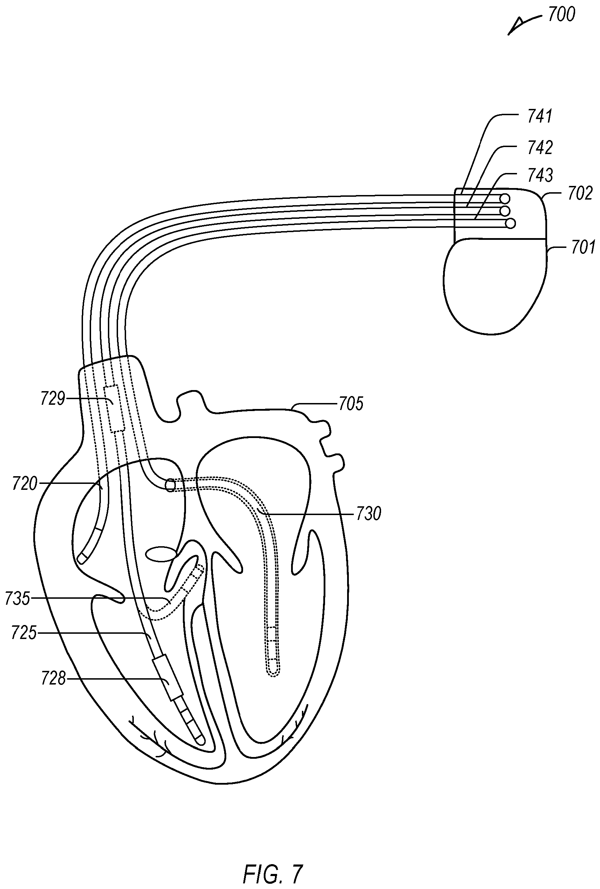

FIG. 7 illustrates an example implantable medical device (IMD) 700 electrically coupled to a heart 705, such as through one or more leads coupled to the implantable medical device 700 through one or more lead ports, such as first, second, or third lead ports 741, 742, 743 in a header 702 of the implantable medical device 700. In an example, the implantable medical device 700 can include an antenna, such as in the header 702, configured to enable communication with an external system and one or more electronic circuits in a hermetically sealed housing (CAN) 701.

The implantable medical device 700 may include an implantable cardiac monitor (ICM), pacemaker, defibrillator, cardiac resynchronizer, or other subcutaneous implantable medical device or cardiac rhythm management (CRM) device configured to be implanted in a chest of a subject, having one or more leads to position one or more electrodes or other sensors at various locations in or near the heart 705, such as in one or more of the atria or ventricles. Separate from, or in addition to, the one or more electrodes or other sensors of the leads, the implantable medical device 700 can include one or more electrodes or other sensors (e.g., a pressure sensor, an accelerometer, a gyroscope, a microphone, etc.) powered by a power source in the implantable medical device 700. The one or more electrodes or other sensors of the leads, the implantable medical device 700, or a combination thereof, can be configured detect physiologic information from, or provide one or more therapies or stimulation to, the patient.

Implantable devices can additionally include a leadless cardiac pacemaker (LCP), small (e.g., smaller than traditional implantable devices, in certain examples having a volume of about 1 cc, etc.), self-contained devices including one or more sensors, circuits, or electrodes configured to monitor physiologic information (e.g., heart rate, etc.) from, detect physiologic conditions (e.g., tachycardia) associated with, or provide one or more therapies or stimulation to the heart 705 without traditional lead or implantable device complications (e.g., required incision and pocket, complications associated with lead placement, breakage, or migration, etc.). In certain examples, a leadless cardiac pacemaker can have more limited power and processing capabilities than a traditional CRM device; however, multiple leadless cardiac pacemaker devices can be implanted in or about the heart to detect physiologic information from, or provide one or more therapies or stimulation to, one or more chambers of the heart. The multiple LCP devices can communicate between themselves, or one or more other implanted or external devices.

The implantable medical device 700 can include one or more electronic circuits configured to sense one or more physiologic signals, such as an electrogram or a signal representing mechanical function of the heart 705. In certain examples, the CAN 701 may function as an electrode such as for sensing or pulse delivery. For example, an electrode from one or more of the leads may be used together with the CAN 701 such as for unipolar sensing of an electrogram or for delivering one or more pacing pulses. A defibrillation electrode (e.g., the first defibrillation coil electrode 728, the second defibrillation coil electrode 729, etc.) may be used together with the CAN 701 to deliver one or more cardioversion/defibrillation pulses.

In an example, the implantable medical device 700 can sense impedance such as between electrodes located on one or more of the leads or the CAN 701. The implantable medical device 700 can be configured to inject current between a pair of electrodes, sense the resultant voltage between the same or different pair of electrodes, and determine impedance, such as using Ohm's Law. The impedance can be sensed in a bipolar configuration in which the same pair of electrodes can be used for injecting current and sensing voltage, a tripolar configuration in which the pair of electrodes for current injection and the pair of electrodes for voltage sensing can share a common electrode, or tetrapolar configuration in which the electrodes used for current injection can be distinct from the electrodes used for voltage sensing, etc. In an example, the implantable medical device 700 can be configured to inject current between an electrode on one or more of the first, second, third, or fourth leads 720, 725, 730, 735 and the CAN 701, and to sense the resultant voltage between the same or different electrodes and the CAN 701.

The implantable medical device 700 can integrate one or more other physiologic sensors to sense one or more other physiologic signals, such as one or more of heart rate, heart rate variability, intrathoracic impedance, intracardiac impedance, arterial pressure, pulmonary artery pressure, RV pressure, LV coronary pressure, coronary blood temperature, blood oxygen saturation, one or more heart sounds, physical activity or exertion level, physiologic response to activity, posture, respiration, body weight, or body temperature. The arrangement and functions of these leads and electrodes are described above by way of example and not by way of limitation. Depending on the need of the patient and the capability of the implantable device, other arrangements and uses of these leads and electrodes are.

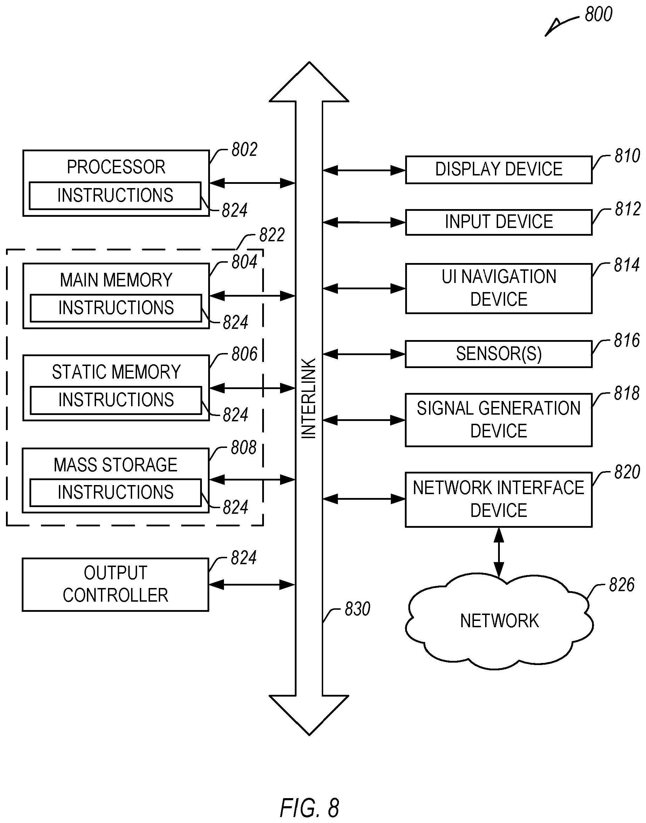

FIG. 8 illustrates a block diagram of an example machine 800 upon which any one or more of the techniques (e.g., methodologies) discussed herein may perform. Portions of this description may apply to the computing framework of one or more of the medical devices described herein, such as the implantable medical device, the external programmer, etc. Further, as described herein with respect to medical device components, systems, or machines, such may require regulatory-compliance not capable by generic computers, components, or machinery.

Examples, as described herein, may include, or may operate by, logic or a number of components, or mechanisms in the machine 800. Circuitry (e.g., processing circuitry, an assessment circuit, etc.) is a collection of circuits implemented in tangible entities of the machine 800 that include hardware (e.g., simple circuits, gates, logic, etc.). Circuitry membership may be flexible over time. Circuitries include members that may, alone or in combination, perform specified operations when operating. In an example, hardware of the circuitry may be immutably designed to carry out a specific operation (e.g., hardwired). In an example, the hardware of the circuitry may include variably connected physical components (e.g., execution units, transistors, simple circuits, etc.) including a machine-readable medium physically modified (e.g., magnetically, electrically, moveable placement of invariant massed particles, etc.) to encode instructions of the specific operation. In connecting the physical components, the underlying electrical properties of a hardware constituent are changed, for example, from an insulator to a conductor or vice versa. The instructions enable embedded hardware (e.g., the execution units or a loading mechanism) to create members of the circuitry in hardware via the variable connections to carry out portions of the specific operation when in operation. Accordingly, in an example, the machine-readable medium elements are part of the circuitry or are communicatively coupled to the other components of the circuitry when the device is operating. In an example, any of the physical components may be used in more than one member of more than one circuitry. For example, under operation, execution units may be used in a first circuit of a first circuitry at one point in time and reused by a second circuit in the first circuitry, or by a third circuit in a second circuitry at a different time. Additional examples of these components with respect to the machine 800 follow.

In alternative embodiments, the machine 800 may operate as a standalone device or may be connected (e.g., networked) to other machines. In a networked deployment, the machine 800 may operate in the capacity of a server machine, a client machine, or both in server-client network environments. In an example, the machine 800 may act as a peer machine in peer-to-peer (P2P) (or other distributed) network environment. The machine 800 may be a personal computer (PC), a tablet PC, a set-top box (STB), a personal digital assistant (PDA), a mobile telephone, a web appliance, a network router, switch or bridge, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

The machine (e.g., computer system) 800 may include a hardware processor 802 (e.g., a central processing unit (CPU), a graphics processing unit (GPU), a hardware processor core, or any combination thereof), a main memory 804, a static memory (e.g., memory or storage for firmware, microcode, a basic-input-output (BIOS), unified extensible firmware interface (UEFI), etc.) 806, and mass storage 808 (e.g., hard drive, tape drive, flash storage, or other block devices) some or all of which may communicate with each other via an interlink (e.g., bus) 830. The machine 800 may further include a display unit 810, an alphanumeric input device 812 (e.g., a keyboard), and a user interface (UI) navigation device 814 (e.g., a mouse). In an example, the display unit 810, input device 812, and UI navigation device 814 may be a touch screen display. The machine 800 may additionally include a signal generation device 818 (e.g., a speaker), a network interface device 820, and one or more sensors 816, such as a global positioning system (GPS) sensor, compass, accelerometer, or one or more other sensors. The machine 800 may include an output controller 824, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc.).

Registers of the processor 802, the main memory 804, the static memory 806, or the mass storage 808 may be, or include, a machine-readable medium 822 on which is stored one or more sets of data structures or instructions 824 (e.g., software) embodying or utilized by any one or more of the techniques or functions described herein. The instructions 824 may also reside, completely or at least partially, within any of registers of the processor 802, the main memory 804, the static memory 806, or the mass storage 808 during execution thereof by the machine 800. In an example, one or any combination of the hardware processor 802, the main memory 804, the static memory 806, or the mass storage 808 may constitute the machine-readable medium 822. While the machine-readable medium 822 is illustrated as a single medium, the term "machine-readable medium" may include a single medium or multiple media (e.g., a centralized or distributed database, and/or associated caches and servers) configured to store the one or more instructions 824.

The term "machine-readable medium" may include any medium that is capable of storing, encoding, or carrying instructions for execution by the machine 800 and that cause the machine 800 to perform any one or more of the techniques of the present disclosure, or that is capable of storing, encoding, or carrying data structures used by or associated with such instructions. Nonlimiting machine-readable medium examples may include solid-state memories, optical media, magnetic media, and signals (e.g., radio frequency signals, other photon-based signals, sound signals, etc.). In an example, a non-transitory machine-readable medium comprises a machine-readable medium with a plurality of particles having invariant (e.g., rest) mass, and thus are compositions of matter. Accordingly, non-transitory machine-readable media are machine-readable media that do not include transitory propagating signals. Specific examples of non-transitory machine-readable media may include: non-volatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magnetooptical disks; and CD-ROM and DVD-ROM disks.

The instructions 824 may be further transmitted or received over a communications network 826 using a transmission medium via the network interface device 820 utilizing any one of a number of transfer protocols (e.g., frame relay, internet protocol (IP), transmission control protocol (TCP), user datagram protocol (UDP), hypertext transfer protocol (HTTP), etc.). Example communication networks may include a local area network (LAN), a wide area network (WAN), a packet data network (e.g., the Internet), mobile telephone networks (e.g., cellular networks), Plain Old Telephone (POTS) networks, and wireless data networks (e.g., Institute of Electrical and Electronics Engineers (IEEE) 802.11 family of standards known as Wi-Fi^{®}, IEEE 802.16 family of standards known as WiMax^{®}), IEEE 802.15.4 family of standards, peer-to-peer (P2P) networks, among others. In an example, the network interface device 820 may include one or more physical jacks (e.g., Ethernet, coaxial, or phone jacks) or one or more antennas to connect to the communications network 826. In an example, the network interface device 820 may include a plurality of antennas to wirelessly communicate using at least one of single-input multiple-output (SIMO), multiple-input multiple-output (MIMO), or multiple-input single-output (MISO) techniques. The term "transmission medium" shall be taken to include any intangible medium that is capable of storing, encoding, or carrying instructions for execution by the machine 800, and includes digital or analog communications signals or other intangible medium to facilitate communication of such software. A transmission medium is a machine-readable medium.

Various embodiments are illustrated in the figures above. One or more features from one or more of these embodiments may be combined to form other embodiments. Method examples described herein can be machine or computer-implemented at least in part. Some examples may include a computer-readable medium or machine-readable medium encoded with instructions operable to configure an electronic device or system to perform methods as described in the above examples. An implementation of such methods can include code, such as microcode, assembly language code, a higher-level language code, or the like. Such code can include computer readable instructions for performing various methods. The code can form portions of computer program products. Further, the code can be tangibly stored on one or more volatile or non-volatile computer-readable media during execution or at other times.

## Claims

1. A medical device system (500) comprising:
a signal receiver circuit (502) configured to receive physiologic information from a patient; and
an assessment circuit (503) configured to:
determine a risk value for each of a plurality of physiologic measures, the plurality of physiologic measures determined using the received physiologic information; and
determine a mortality risk metric indicative of a risk of patient mortality as a weighted combination of the plurality of physiologic measures having risk values satisfying a pre-determined condition,
wherein the plurality of physiologic measures include one or more of impedance information, activity information, respiratory rate information, and heart sound information,
wherein the risk value includes a categorical risk value, the categorical risk value indicating one of a high risk or a low risk,
wherein the pre-determined condition comprises a determined indicating the high risk,
wherein the assessment circuit (503) is configured to adjust a weighting or the combination of the weighted combination of physiologic measures based on the determined categorical risk values indicating the high risk,
wherein the heart sound information comprises a combination of third heart sound (S3) information and first heart sound (S1) information,
wherein the combination of S3 information and S1 information comprises a ratio of S3/S1.

2. The medical device system (500) of claim 1, wherein the assessment circuit (503) is configured to adjust a weighting or combination of the weighted combination of the plurality of physiologic measures based on a combination of third heart sound (S3) information, the respiratory rate information, and the activity information,
wherein the combination of physiologic measures includes at least two of first heart sound (S1) information, the third heart sound (S3) information, the impedance information, the respiratory rate information, or the activity information.

3. The medical device system (500) of any one of claims 1 through 2, wherein the signal receiver circuit (502) is configured to receive clinical information about the patient separate from the received physiologic information, the clinical information including at least one of: patient demographic information; diagnosed comorbidities; previous treatment or hospitalization; or a type of implanted device,
wherein the assessment circuit (503) is configured to determine the mortality risk metric indicative of the risk of patient mortality using the weighted combination of the plurality of physiologic measures and the received clinical information about the patient separate from the received physiologic information.

4. The medical device system (500) of any one of claims 1 through 3, wherein the assessment circuit (503) is configured to determine an alert state of the patient using the determined mortality risk metric and a threshold, and to further adjust the determined mortality risk metric as a function of a time that the determined mortality risk metric is above the threshold.

5. The medical device system (500) of any one of claims 1 through 4, wherein the assessment circuit (503) is configured to schedule determinations of the mortality risk metric at a default time period, and to adjust the default time period as a function of a value of the determined mortality risk metric.

6. The medical device system (500) of any one of claims 1 through 5, wherein the system comprises an implantable medical device (602) comprising the assessment circuit (503),
wherein the assessment circuit (503) is configured to alter or adjust one or more modes or functions of the implantable medical device (602) based on the determined mortality risk metric,
wherein the one or more modes or functions includes at least one of: an active state of a sensor (501) of the implantable medical device (602); a sampling frequency or resolution of a sensor (501) of the implantable medical device (602); an amount of data storage of physiologic information; or a time of communication of stored information outside of the implantable medical device (602).

7. The medical device system (500) of any one of claims 1 through 6, wherein the assessment circuit (503) is configured to determine a trend of the determined mortality risk metric over time and to provide an output of the determined mortality risk metric trend to a user interface for display to a user or to another circuit to control or adjust a process or function of the system.

8. The medical device system (500) of any one of claims 1 through 7, wherein the assessment circuit (503) configured to determine the mortality risk metric indicative of the risk of patient mortality as a relative difference between respective determined mortality risk metrics at different times.

9. The medical device system (500) of any one of claims 1 through 8, wherein the system comprises an implantable medical device (602) comprising the assessment circuit (503),
wherein the assessment circuit (503) is configured to determine an estimated remaining battery status of the implantable medical device (602),
wherein the assessment circuit (503) is configured to alter or adjust one or more modes or functions of the implantable medical device (602) based on a difference between the determined mortality risk metric and the determined estimated remaining battery status of the implantable medical device (602).

10. The medical device system (500) of any one of claims 1 through 9, wherein the assessment circuit (503) is configured to determine the mortality risk metric using a weighted combination of third heart sound (S3) information and first heart sound (S1) information,
wherein the assessment circuit (503) is configured to adjust a weighting or combination of the weighted combination based on a combination of the S3 information, the respiration information, and the activity information.

11. A method comprising:
receiving, a signal receiver circuit (502), physiologic information from a patient;
determining, using an assessment circuit (503), a risk value for each of a plurality of physiologic measures, the plurality of physiologic measures determined using the received physiologic information; and
determining, using the assessment circuit (503), a mortality risk metric indicative of a risk of patient mortality using a weighted combination of the plurality of physiological measures having determined risk values satisfying a condition,
wherein the plurality of physiologic measures include one or more of impedance information, activity information, respiratory rate information, and heart sound information,
wherein the risk value includes a categorical risk value, the categorical risk value indicating one of a high risk or a low risk,
wherein the pre-determined condition comprises a determined indicating the high risk,
wherein the method comprises adjusting a weighting or the combination of the weighted combination of physiologic measures based on the determined categorical risk values indicating the high risk,
wherein the heart sound information comprises a combination of third heart sound (S3) information and first heart sound (S1) information,
wherein the combination of S3 information and S1 information comprises a ratio of S3/S1.

## Patentansprüche

1. Medizinisches Vorrichtungssystem (500), umfassend:
Signalempfängerschaltung (502), die eingerichtet ist, physiologische Informationen von einem Patienten zu empfangen; und
eine Beurteilungsschaltung (503), die eingerichtet ist zum:
Bestimmen eines Risikowerts für jeden einer Vielzahl von physiologischen Meßwerten, wobei die Vielzahl von physiologischen Meßwerten anhand der empfangenen physiologischen Informationen bestimmt wird; und
Bestimmen einer Sterberisikometrik, die das Risiko eines Patiententodes widerspiegelt, und zwar als gewichtete Kombination der Vielzahl von physiologischen Messwerten, deren Risikowerte eine vorab festgelegte Bedingung erfüllen,
wobei die Vielzahl physiologischer Messwerte eine oder mehrere der folgenden Informationen umfasst: Impedanzinformationen, Aktivitätsinformationen, Atemfrequenzinformationen und Herzgeräuschinformationen,
wobei der Risikowert einen kategorialen Risikowert umfasst, wobei der kategoriale Risikowert entweder ein hohes oder ein geringes Risiko angibt,
wobei die vorbestimmte Bedingung einen Indikator umfasst, der auf ein hohes Risiko hinweist,
wobei die Beurteilungsschaltung (503) eingerichtet ist, eine Gewichtung oder die Kombination der gewichteten Kombination physiologischer Messwerte auf der Grundlage der bestimmten kategorialen Risikowerte, die auf ein hohes Risiko hinweisen, anzupassen,
wobei die Herzgeräuschinformationen eine Kombination aus Informationen zum dritten Herzgeräusch (S3) und Informationen zum ersten Herzgeräusch (S1) umfassen,
wobei die Kombination aus S3-Informationen und S1-Informationen ein Verhältnis von S3 zu S1 umfasst.

2. Medizinisches Vorrichtungssystem (500) nach Anspruch 1, wobei die Beurteilungsschaltung (503) eingerichtet ist, eine Gewichtung oder die Kombination der gewichteten Kombination der Vielzahl von physiologischen Messwerten auf der Grundlage einer Kombination aus Informationen zum dritten Herzton (S3), Atemfrequenzinformationen und Aktivitätsinformationen anzupassen,
wobei die Kombination physiologischer Messwerte mindestens zwei der folgenden Größen umfasst: Informationen zum ersten Herzton (S1), Informationen zum dritten Herzton (S3), Impedanzinformationen, Atemfrequenzinformationen oder Aktivitätsinformationen.

3. Medizinisches Vorrichtungssystem (500) nach einem der Ansprüche 1 bis 2, wobei die Signalempfängerschaltung (502) eingerichtet ist, klinische Informationen über den Patienten getrennt von den empfangenen physiologischen Informationen zu empfangen, wobei die klinischen Informationen mindestens eines der folgenden Elemente umfassen: demographische Informationen über den Patienten; diagnostizierte Begleiterkrankungen; frühere Behandlungen oder Krankenhausaufenthalte; oder einen Typ einer implantierten Vorrichtung,
wobei die Beurteilungsschaltung (503) eingerichtet ist, die das Risiko des Patiententodes wiederspiegelnde Sterberisikometrik unter Verwendung der gewichteten Kombination aus der Vielzahl von physiologischen Messwerten und den getrennt von den empfangenen physiologischen Informationen empfangenen klinischen Informationen über den Patienten zu bestimmen.

4. Medizinisches Vorrichtungssystem (500) nach einem der Ansprüche 1 bis 3, wobei die Beurteilungsschaltung (503) eingerichtet ist, anhand der bestimmten Sterberisikometrik und eines Schwellenwerts einen Alarmzustand des Patienten zu bestimmen und die bestimmte Sterberisikometrik in Abhängigkeit von der Zeit, während der die bestimmte Sterberisikometrik über dem Schwellenwert liegt, weiter anzupassen.

5. Medizinisches Vorrichtungssystem (500) nach einem der Ansprüche 1 bis 4, wobei die Beurteilungsschaltung (503) eingerichtet ist, das Bestimmen der Sterberisikometrik in einem Standardzeitraum zu planen und den Standardzeitraum in Abhängigkeit von einem Wert der bestimmten Sterberisikometrik anzupassen.

6. Medizinische Vorrichtung (500) nach einem der Ansprüche 1 bis 5, wobei das System eine implantierbare medizinische Vorrichtung (602) umfasst, die die Beurteilungsschaltung (503) enthält,
wobei die Beurteilungsschaltung (503) eingerichtet ist, einen oder mehrere Betriebsmodi oder Funktionen der implantierbaren medizinischen Vorrichtung (602) auf der Grundlage der bestimmten Sterberisikometrik zu ändern oder anzupassen,
wobei der eine oder die mehreren Modi oder Funktionen mindestens eines der folgenden Elemente umfassen: einen aktiven Zustand eines Sensors (501) der implantierbaren medizinischen Vorrichtung (602); eine Abtastfrequenz oder Auflösung eines Sensors (501) der implantierbaren medizinischen Vorrichtung (602); einen Umfang der Datenspeicherung physiologischer Informationen; oder einen Zeitpunkt der Übertragung gespeicherter Informationen außerhalb der implantierbaren medizinischen Vorrichtung (602).

7. Medizinisches Vorrichtungssystem (500) nach einem der Ansprüche 1 bis 6, wobei die Beurteilungsschaltung (503) eingerichtet ist, einen Trend der bestimmten Sterberisikometrik im Zeitverlauf zu bestimmen und den bestimmten Trend der Sterberisikometrik an eine Benutzerschnittstelle zur Anzeige für einen Benutzer oder an eine andere Schaltung zur Steuerung oder Anpassung eines Prozesses oder einer Funktion des Systems auszugeben.

8. Medizinisches Vorrichtungssystem (500) nach einem der Ansprüche 1 bis 7, wobei die Beurteilungsschaltung (503) eingerichtet ist, die das Risiko des Patientendodes wiederspiegelnde Sterberisikometrik als relative Differenz zwischen jeweils zu unterschiedlichen Zeitpunkten bestimmten Sterberisikometriken zu bestimmen.

9. Medizinisches Vorrichtungssystem (500) nach einem der Ansprüche 1 bis 8, wobei das System eine implantierbare medizinische Vorrichtung (602) umfasst, die die Beurteilungsschaltung (503) umfasst,
wobei die Beurteilungsschaltung (503) eingerichtet ist, einen geschätzten verbleibenden Batteriestatus der implantierbaren medizinischen Vorrichtung (602) zu bestimmen,
wobei die Beurteilungsschaltung (503) eingerichtet ist, einen oder mehrere Betriebsmodi oder Funktionen der implantierbaren medizinischen Vorrichtung (602) auf der Grundlage einer Differenz zwischen der bestimmten Sterberisikometrik und dem bedstimmten geschätzten verbleibenden Batteriestatus der implantierbaren medizinischen Vorrichtung (602) zu ändern oder anzupassen.

10. Medizinisches Vorrichtungssystem (500) nach einem der Ansprüche 1 bis 9, wobei die Beurteilungsschaltung (503) eingerichtet ist, die Sterberisikometrik anhand einer gewichteten Kombination aus Informationen zum dritten Herzton (S3) und Informationen zum ersten Herzton (S1) zu bestimmen,
wobei die Beurteilungsschaltung (503) eingerichtet ist, eine Gewichtung oder die Kombination der gewichteten Kombination auf der Grundlage einer Kombination aus den S3-Informationen, den Respirationsinformationen und den Aktivitätsinformationen anzupassen.

11. Verfahren, umfassend:
Empfangen physiologischer Informationen von einem Patienten mittels einer Signalempfängerschaltung (502);
Bestimmen eines Risikowerts für jeden einer Vielzahl von physiologischen Messwerten unter Verwendung einer Beurteilungsschaltung (503), wobei die Vielzahl von physiologischen Messwerten anhand der empfangenen physiologischen Informationen bestimmt wird; und
Bestimmen einer Sterberisikometrik, die ein Risiko des Patiententodes wiederspiegelt, unter Verwendung der Beurteilungsschaltung (503) und unter Verwendung einer gewichteten Kombination der Vielzahl von physiologischen Messwerten, deren ermittelte Risikowerte eine Bedingung erfüllen,
wobei die Vielzahl physiologischer Messwerte eine oder mehrere der folgenden Informationen umfasst: Impedanzinformationen, Aktivitätsinformationen, Atemfrequenzinformationen und Herzgeräuschinformationen,
wobei der Risikowert einen kategorialen Risikowert umfasst, wobei der kategoriale Risikowert entweder ein hohes oder ein geringes Risiko angibt,
wobei die vorbestimmte Bedingung einen Indikator umfasst, der auf ein hohes Risiko hinweist,
wobei das Verfahren das Anpassen einer Gewichtung oder der Kombination der gewichteten Kombination physiologischer Messwerte auf der Grundlage der ermittelten kategorialen Risikowerte umfasst, die auf ein hohes Risiko hinweisen,
wobei die Herzgeräuschinformationen eine Kombination aus Informationen zum dritten Herzgeräusch (S3) und Informationen zum ersten Herzgeräusch (S1) umfassen,
wobei die Kombination aus S3-Informationen und S1-Informationen ein Verhältnis von S3 zu S1 umfasst.

## Revendications

1. Système de dispositif médical (500) comprenant :
un circuit de récepteur de signaux (502) configuré pour recevoir des informations physiologiques en provenance d'un patient ; et
un circuit d'évaluation (503) configuré pour :
déterminer une valeur de risque pour chacun d'une pluralité de paramètres physiologiques mesurés, les paramètres de la pluralité de paramètres physiologiques mesurés étant déterminés en utilisant les informations physiologiques reçues ; et pour :
déterminer un indice de risque de mortalité qui est indicatif d'un risque de mortalité du patient en tant que combinaison pondérée de la pluralité de paramètres physiologiques mesurés dont les valeurs de risque satisfont une condition prédéterminée,
dans lequel : la pluralité de paramètres physiologiques mesurés inclut un ou plusieurs ensembles d'informations parmi des informations relatives à l'impédance, des informations relatives à l'activité, des informations relatives à la fréquence respiratoire et des informations relatives aux bruits du cœur,
dans lequel : la valeur de risque inclut une valeur de risque catégorielle, la valeur de risque catégorielle indiquant soit un risque élevé, soit un risque faible,
dans lequel : la condition prédéterminée correspond à une détermination qui indique un risque élevé,
dans lequel : le circuit d'évaluation (503) est configuré pour ajuster une pondération ou la composition combinatoire de la combinaison pondérée de paramètres physiologiques mesurés sur la base des valeurs de risque catégorielles déterminées qui indiquent le risque élevé,
dans lequel : les informations relatives aux bruits du cœur comprennent une combinaison d'informations relatives au troisième bruit du cœur (S3) et d'informations relatives au premier bruit du cœur (S1), et
dans lequel : la combinaison des informations relatives à S3 et des informations relatives à S1 comprend un rapport S3/S1.

2. Système de dispositif médical (500) selon la revendication 1, dans lequel : le circuit d'évaluation (503) est configuré pour ajuster une pondération ou composition combinatoire de la combinaison pondérée de la pluralité de paramètres physiologiques mesurés sur la base d'une combinaison des informations relatives au troisième bruit du cœur (S3), des informations relatives à la fréquence respiratoire et des informations relatives à l'activité, et
dans lequel : la combinaison de paramètres physiologiques mesurés inclut au moins deux ensembles d'informations parmi les informations relatives au premier bruit du cœur (S1), les informations relatives au troisième bruit du cœur (S3), les informations relatives à l'impédance, les informations relatives à la fréquence respiratoire et les informations relatives à l'activité.

3. Système de dispositif médical (500) selon l'une quelconque des revendications 1 et 2, dans lequel : le circuit de récepteur de signaux (502) est configuré pour recevoir des informations cliniques qui concernent le patient, lesquelles informations cliniques sont distinctes des informations physiologiques reçues, les informations cliniques incluant au moins un ensemble d'informations parmi des informations démographiques du patient ; des comorbidités diagnostiquées ; des traitements antérieurs ou des hospitalisations antérieures ; et un type de dispositif implanté, et
dans lequel : le circuit d'évaluation (503) est configuré pour déterminer l'indice de risque de mortalité qui est indicatif du risque de mortalité du patient en utilisant la combinaison pondérée de la pluralité de paramètres physiologiques mesurés et les informations cliniques reçues qui concernent le patient, distinctes des informations physiologiques reçues.

4. Système de dispositif médical (500) selon l'une quelconque des revendications 1 à 3, dans lequel le circuit d'évaluation (503) est configuré pour déterminer un état d'alerte du patient en utilisant l'indice de risque de mortalité déterminé et une valeur de seuil, et pour en outre ajuster l'indice de risque de mortalité déterminé en fonction d'une durée pendant laquelle l'indice de risque de mortalité déterminé est au-delà de la valeur de seuil.

5. Système de dispositif médical (500) selon l'une quelconque des revendications 1 à 4, dans lequel le circuit d'évaluation (503) est configuré pour effectuer de façon planifiée des déterminations de l'indice de risque de mortalité selon une période temporelle par défaut et pour ajuster la période temporelle par défaut en fonction d'une valeur de l'indice de risque de mortalité déterminé.

6. Système de dispositif médical (500) selon l'une quelconque des revendications 1 à 5, dans lequel : le système comprend un dispositif médical implantable (602) qui comprend le circuit d'évaluation (503),
dans lequel : le circuit d'évaluation (503) est configuré pour modifier ou ajuster un ou plusieurs modes de fonctionnement ou une ou plusieurs fonctions du dispositif médical implantable (602) sur la base de l'indice de risque de mortalité déterminé, et
dans lequel : les un ou plusieurs modes de fonctionnement ou les une ou plusieurs fonctions incluent au moins une information parmi un état actif d'un capteur (501) du dispositif médical implantable (602) ; une fréquence ou résolution d'échantillonnage d'un capteur (501) du dispositif médical implantable (602) ; une quantité d'informations physiologiques stockées en tant que données ; et un moment de communication des informations physiologiques stockées à l'extérieur du dispositif médical implantable (602).

7. Système de dispositif médical (500) selon l'une quelconque des revendications 1 à 6, dans lequel le circuit d'évaluation (503) est configuré pour déterminer une évolution tendancielle dans le temps de l'indice de risque de mortalité déterminé et pour fournir un signal de sortie indicatif de l'évolution tendancielle de l'indice de risque de mortalité déterminé à une interface utilisateur dans le but de son affichage pour un utilisateur ou à un autre circuit destiné à commander ou à ajuster un processus ou une fonction du système.

8. Système de dispositif médical (500) selon l'une quelconque des revendications 1 à 7, dans lequel le circuit d'évaluation (503) est configuré pour déterminer l'indice de risque de mortalité qui représente le risque de mortalité du patient en tant que différence relative entre des indices de risque de mortalité déterminés respectifs à des moments différents.

9. Système de dispositif médical (500) selon l'une quelconque des revendications 1 à 8, dans lequel : le système comprend un dispositif médical implantable (602) qui comprend le circuit d'évaluation (503),
dans lequel : le circuit d'évaluation (503) est configuré pour déterminer une estimation de la capacité résiduelle de la batterie du dispositif médical implantable (602), et
dans lequel : le circuit d'évaluation (503) est configuré pour modifier ou ajuster un ou plusieurs modes de fonctionnement ou une ou plusieurs fonctions du dispositif médical implantable (602) sur la base d'une différence entre l'indice de risque de mortalité déterminé et l'estimation déterminée de la capacité résiduelle de la batterie du dispositif médical implantable (602).

10. Système de dispositif médical (500) selon l'une quelconque des revendications 1 à 9, dans lequel : le circuit d'évaluation (503) est configuré pour déterminer l'indice de risque de mortalité en utilisant une combinaison pondérée des informations relatives au troisième bruit du cœur (S3) et des informations relatives au premier bruit du cœur (S1), et
dans lequel : le circuit d'évaluation (503) est en outre configuré pour ajuster une pondération ou composition combinatoire de la combinaison pondérée sur la base d'une combinaison des informations relatives au troisième bruit du cœur (S3), des informations relatives à la fréquence respiratoire et des informations relatives à l'activité.

11. Procédé comprenant :
la réception, en utilisant un circuit de récepteur de signaux (502), d'informations physiologiques en provenance d'un patient ;
la détermination, en utilisant un circuit d'évaluation (503), d'une valeur de risque pour chacun d'une pluralité de paramètres physiologiques mesurés, les paramètres de la pluralité de paramètres physiologiques mesurés étant déterminés en utilisant les informations physiologiques reçues ; et
la détermination, en utilisant le circuit d'évaluation (503), d'un indice de risque de mortalité qui est indicatif d'un risque de mortalité du patient en utilisant une combinaison pondérée de la pluralité de paramètres physiologiques mesurés dont les valeurs de risque déterminées satisfont une condition prédéterminée,
dans lequel : la pluralité de paramètres physiologiques mesurés inclut un ou plusieurs ensembles d'informations parmi des informations relatives à l'impédance, des informations relatives à l'activité, des informations relatives à la fréquence respiratoire et des informations relatives aux bruits du cœur,
dans lequel : la valeur de risque inclut une valeur de risque catégorielle, la valeur de risque catégorielle indiquant soit un risque élevé, soit un risque faible,
dans lequel : la condition prédéterminée correspond à une détermination qui indique un risque élevé,
dans lequel : le procédé comprend l'ajustement d'une pondération ou de la composition combinatoire de la combinaison pondérée de paramètres physiologiques mesurés sur la base des valeurs de risque catégorielles déterminées qui indiquent le risque élevé,
dans lequel : les informations relatives aux bruits du cœur comprennent une combinaison d'informations relatives au troisième bruit du cœur (S3) et d'informations relatives au premier bruit du cœur (S1), et
dans lequel : la combinaison des informations relatives à S3 et des informations relatives à S1 comprend un rapport S3/S1.
